(19) Europäisches Patentamt
European Patent Office
Office européen des brevets

(11) Publication number: **0 114 563**
**A1**

(12) # EUROPEAN PATENT APPLICATION

(21) Application number: 83730119.1

(22) Date of filing: 09.12.83

(51) Int. Cl.³: A 61 K 31/48
//(A61K31/48, 31/195)

(30) Priority: 20.12.82 DE 3247514

(43) Date of publication of application:
01.08.84 Bulletin 84/31

(84) Designated Contracting States:
AT BE CH DE FR GB IT LI LU NL SE

(71) Applicant: SCHERING AKTIENGESELLSCHAFT Berlin und Bergkamen
Müllerstrasse 170/178 Postfach 65 03 11
D-1000 Berlin 65(DE)

(72) Inventor: Birkmayer, Jörg, Dr.
Schwarzspanierstrasse 16
A-1090 Wien(AT)

(72) Inventor: Horowski, Reinhard, Dr.
Am Rosenanger 32
D-1000 Berlin 28(DE)

(54) **Cytostatically effective combination.**

(57) A combination of an ergot alkaloid, e.g., lisuride, and L-dopa has synergistic cytostatic activity.

EP 0 114 563 A1

# CYTOSTATICALLY EFFECTIVE COMBINATION

## BACKGROUND OF THE INVENTION

This invention relates to a synergistic combination having cytostatic activity.

It has been known for a long time that ergot alkaloids exhibit pharmacological activity. In recent times, therapeutic uses of several of these have become conventional. For example, bromocriptine (DAS 1,926,045; U.S. Patents 3,752,814 and 3,752,888) is used therapeutically as a lactation inhibitor. Lisuride hydrogen maleate (U.S. Patent 3,681,497) is known as a migraine medicine. Transdihydrolisuride (DAS 2,238,540) acts pharmacologically as a nidation inhibitor, for example.

Furthermore, the use of L-dopa (3-hydroxy-L-tyrosine) as an agent against parkinsonism has been known for some time.

## SUMMARY OF THE INVENTION

It is an object of this invention to provide a new combination of these known agents having new valuable pharmacological activity.

Upon further study of the specification and appended claims, further objects and advantages of this invention will become apparent to those skilled in the art.

Preferably, the ergot alkaloid is an ergoline derivative of the formula

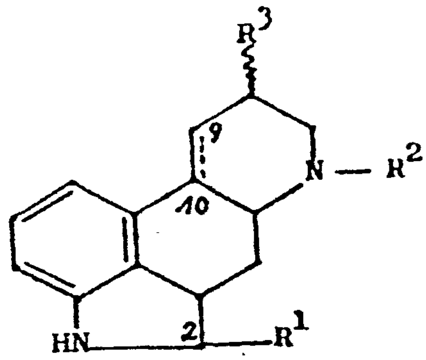

wherein

$R^1$ is hydrogen, chlorine, bromine, or iodine,

$R^2$ is hydrogen or $C_{1-6}$-alkyl,

$R^3$ is $-CH_2-CN$, $-CH_2-S-CH_3$, $-NH-SO_2NR'R''$, $-NH-CS-NR'R''$, $-NH-CO-NR'R''$ ($R'$, $R''$ = H or $C_{1-6}$-alkyl), or

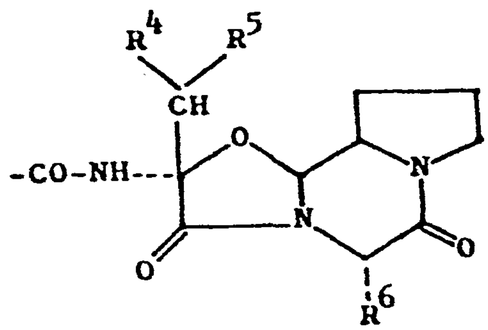

$R^4$ and $R^5$ each independently is hydrogen or methyl and $R^6$ is phenylmethyl, isobutyl, or isopropyl, and $C_9$----$C_{10}$ is a CC single or double bond.

Also preferred are synergistic combinations wherein the ergot alkaloid and L-dopa are used in a weight ratio of from 1:10 to 10:1, especially about 1:1, for the pharmacological studies, whereas the ratio is different when used in human medicine.

DETAILED DISCUSSION

Suitable alkyl groups for $R^2$, R' and R" include methyl, ethyl, n-propyl, isopropyl, n-butyl, isobutyl, sec-butyl, t-butyl, pentyl, isopentyl, hexyl, isohexyl, etc.

Examples of suitable ergot alkaloids include lisuride hydrogen maleate, 1,1-diethyl-3-(6-methyl-8α-ergolinyl)urea, 1,1-diethyl-3-(2-bromo-6-methyl-8α-ergolinyl)urea, 1,1-diethyl-3-(2-bromo-6-methyl-9,10-didehydro-8α-ergolinyl)urea, 1,1-diethyl-3-(9,10-didehydro-6-n-propyl-8α-ergolinyl)urea, 1,1-diethyl-3-(6-n-propyl-8α-ergolinyl)urea, pergolide, lergotril, bromocriptine, or ergotamine and the like.

All known ergot alkaloids and their derivatives having known pharmacological activity based upon high affinity for monoamine and particularly for dopamine receptors can be used in the combination of this invention, e.g., those described in ERGOT ALKALOIDS and RELATED COMPOUNDS, B. Berde and H.O. Schild, Editors, Handbook of Experimental Pharmacology, Springer Berlin, 1978.

The ergot alkaloids used in this invention can be in the form of the free base or in the form of one of the conventional pharmacologically acceptable addition salts. Such addition salts are preferably derived from physiologically compatible acids, for example, tartaric, maleic, methanesulfonic, phosphoric, hydrochloric acids and the many other well known acids used to form such salts.

The following investigations have been conducted in conjunction with this invention.

Human leukemic cells, lung carcinoma and mamma carcinoma cells were grown in vitro with a culture medium (minimally essential medium with 10% fetal calf serum) to which were separately added lisuride by itself, L-dopa by itself, and combinations of lisuride

and L-dopa in various amounts. The growth kinetics, i.e., the proliferation rate per unit time, was determined by counting the number of cells after 48, 72, 96, and 144 hours and compared with that of cells cultivated in a lisuride-free medium with addition of no L-dopa. Every second day, the culture medium of each test batch was replaced by fresh medium and active agent.

An inhibition of cell proliferation was observed for this invention, clearly demonstrating the synergistic influence of L-dopa.

| Time [Hrs.] | Lisuride 20 µg/ml | L-Dopa 10 µg/ml | Lisuride + L-Dopa |
|---|---|---|---|
| 72 | 17.0 % | 18.0 % | 44.0 % |
| 144 | 36.5 % | 58.0 % | 78.0 % |

**The above results were obtained by using human leukemic cells.**

The numbers in the table indicate the inhibition in %, based on a control free of active agent.

It was definitely surprising that the combination of an ergot alkaloid with L-dopa would inhibit or stop growth and proliferation of malignant cells, such as those of human leukemia, carcinoma of the lung, and mamma carcinoma. In connection with the tumor cells, the combination has a synergistic effect and permits the use of lower concentrations and/or dosages of the individual compounds. These results thus cause lesser cytotoxicity in healthy cells and fewer side effects, respectively. Such a combination in other instances would almost always be senseless.

Theoretically, the sum total effect of the dopaminergic effect of the ergot alkaloids and the dopamine produced from L-dopa should not differ from the effect of correspondingly higher quantities of the individual compounds.

Consequently, the combination of this invention is suitable for the treatment of leukemia and all solid human tumors such as carcinomas and sarcomas.

However, in particular, this combination is suited for the treatment of leukemias and carcinomas which are related to the systems tested, e.g., neuroblastomas, carcinomas of the thyroid and adrenal gland, and of carcinomas of the bronchi, lung, and mamma (breast).

The combination of this invention, as compared with known cytostatic agents, such as endoxan or methotrexate which act as growth inhibitors for all dividing cells, exhibits the advantage that the considerable side effects occurring with these known agents, e.g., disturbances in blood formation and in the gastrointestinal tract, loss of hair, spermiogenesis disturbances, etc., are eliminated. This is also confirmed by this invention's lack of growth-inhibiting effects on normal, nontumorous cells in a culture, and also by the fact that none of these effects were encountered individually for the above-indicated components of the combination during the conductance of long-term compatibility and carcinogenicity tests on animals and also in a broad-scale clinical investigation on human subjects.

The combination of this invention can thus be utilized, on account of this lack of side effects,

also for therapies reaching far beyond the customarily maintained 3 months, and likewise in patients unable to tolerate the known side effects of cytostatic agents, such as weakness of the body's defenses, anemia, tendency to hemorrhage, etc. The use of L-dopa in combination with an ergot alkaloid furthermore has the advantage that each compound can be used in lower doses than in the case of therapy with each drug separately. Therefore, the side effects which are observed can be reduced.

The active agents of the combination of this invention are administered in the usual form of pharmaceutical preparations to humans. For example, the pharmaceutical preparation can be utilized for oral administration in the form of tablets, powders, granules, capsules, syrups, and elixirs, and also in the form of solutions, suspensions, dispersions, and emulsions for parenteral administration, e.g., of a sterile injectable aqueous solution. The preparations suitable for oral administration can contain the customary excipients and auxiliary agents, wherein the active agents can also be microencapsulated, for example, for depot formulations.

Conventional excipients are pharmaceutically acceptable organic or inorganic carrier substances suitable for parenteral, enteral or topical application which do not deleteriously react with the active compounds. Suitable pharmaceutically acceptable carriers include but are not limited to water, salt solutions, alcohols, gum arabic, vegetable oils, polyethylene glycols, gelatine, lactose, amylose, magnesium stearate, talc, silicic acid, viscous paraffin, perfume oil, fatty acid monoglycerides and diglycerides, pentaerythritol fatty acid esters, hydroxy-methylcellulose, polyvinyl pyrrolidone, etc. The pharmaceutical preparations can be sterilized and if desired mixed with auxiliary

agents, e.g., lubricants, preservatives, stabilizers, wetting agents, emulsifiers, salts for influencing osmotic pressure, buffers, coloring, flavoring and/or aromatic substances and the like which do not deleteriously react with the active compounds.

For parenteral application, particularly suitable are injectable sterile solutions, preferably oily or aqueous solutions, as well as suspensions, emulsions, or implants, including suppositories. Ampoules are convenient unit dosages.

For enteral application, particularly suitable are tablets, dragees, suppositories or capsules having talc and/or a carbohydrate carrier or binder or the like, the carrier preferably being lactose and/or corn starch and/or potato starch. A syrup, elixir or the like can be used wherein a sweetened vehicle is employed. Sustained release compositions can be formualted including those wherein the active compound is protected with differentially degradable coatings, e.g., by microencapsulation, multiple coatings, etc.

The compounds of the combination of this invention can be administered individually or together.

The dosage of the combination of this invention to be daily administered naturally depends on the type of case to be treated. Dosages for a given host for a given indication can be determiend, e.g., by customary comparison of the activities of the subject combination and of a known agent by means of an appropriate, conventional pharmacological protocol. When used in human medicine, the amount of the ergot alkaloid in the combination to be administered orally is usually 0.01-50 mg/day, preferably 0.5-10 mg/day. For parenteral administration, for example in long-term therapy, the daily dosage of the ergot alkaloid is 0.15-1.5 mg/day.

Suitable amounts of L-dopa in the combination include for example, 25-1,000 mg (p.o./i.v.) alone or in conventional combination with the usual amount of one of the conventional decarboxylase inhibitors, such as benserazide or carbidopa. If necessary, higher daily doses are also possible.

The proportion of ergot alkaloid in the combination can be largely varied as it depends on the pharmacological activity and the bioavailability of the compounds in humans.

The individual dosage forms contain, per dosage unit, 0.01-50 mg of the ergot alkaloid and 10-500 mg of L-dopa.

From the standpoint of manufacturing considerations and favorable possibilities of administration, injection prepraations in the form of ampoules or solid preparations, especially capsules or tablets, are preferred.

Except as indicated above, e.g., except for the additional latitude provided by the advantageous aspects of this invention as described above, the administration of the combination of this invention is analogous to the combination treatment by conventional cytostatic agents, e.g., use of vincristine, methotrexate and preduisolone in acute leukemia, or of cis-platinum and cyclophosphamide in epithelical tumors.

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure in any way whatsoever. In the following Examples, all temperatures are set forth uncorrected in degrees Celsius; unless otherwise indicated, all parts and percentages are by weight.

Example 1

The tablets suitable for oral administration and containing the ingredients set out below are produced in a conventional process.

| Ingredients | Weight |
|---|---|
| 1,1-Diethyl-3-(6-methyl-8α-ergolinyl)urea dihydrogen phosphate | 1.00 mg |
| L-Dopa | 100.00 mg |
| Lactose | 97.85 mg |
| Corn starch | 14.00 mg |
| Polyvinylpyrrolidone | 5.00 mg |
| Magnesium stearate | 0.70 mg |
| Talc | 1.45 mg |
| | 220.00 mg |

Example 2

The capsules suitable for enteral administration and containing the ingredients disclosed below are manufactured in a manner known per se.

| Ingredients | Weight |
|---|---|
| Bromocriptine methanesulfonate | 3.00 mg |
| L-Dopa | 100.00 mg |
| Lactose | 95.00 mg |
| Corn starch | 20.00 mg |
| Talc | 4.50 mg |
| "Aerosil" | 1.00 mg |
| Magnesium stearate | 1.50 mg |
| | 225.00 mg |

Example 3

Analogously to Example 2, tablets are produced having the following composition:

| Ingredients | Weight |
|---|---|
| 1,1-Diethyl-3-(6-methyl-9,10-didehydro-8α-ergolinyl)urea dihydrogen maleate | 5.00 mg |
| L-Dopa | 50.00 mg |
| Lactose | 99.05 mg |
| Corn starch | 19.00 mg |
| Polyvinylpyrrolidone | 5.00 mg |
| Magnesium stearate | 0.70 mg |
| Talc | 1.25 mg |
| | 180.00 mg |

The preceding examples can be repeated with similar success by substituting the generically or specifically described reactants and/or operating conditions of this invention for those used in the preceding examples. From the foregoing description, one skilled in the art can easily ascertain the essential characteristics of this invention, and without departing from the spirit and scope thereof, can make various changes and modifications of the invnetion to adapt it to various usages and conditions.

WHAT IS CLAIMED IS:

1. A pharmaceutical composition comprising a cytostatically effective amount of a combination of an ergot alkaloid and L-dopa.

2. A composition of claim 1, further comprising a pharmacologically acceptable carrier.

3. A composition of claim 1, wherein the ergot alkaloid is an ergoline derivative of the formula

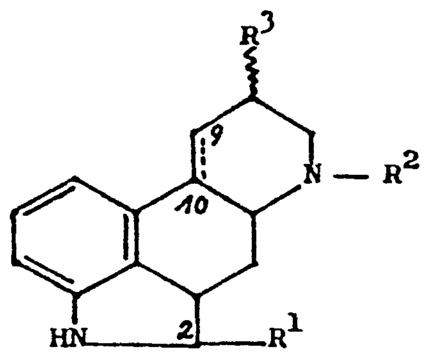

wherein

$R^1$ is hydrogen, chlorine, bromine, or iodine,

$R^2$ is hydrogen or $C_{1-6}$-alkyl,

$R^3$ is $-CH_2-CN$, $-CH_2-S-CH_3$, $-NH-SO_2NR'R''$, $-NH-CS-NR'R''$, $-NH-CO-NR',R''$, or

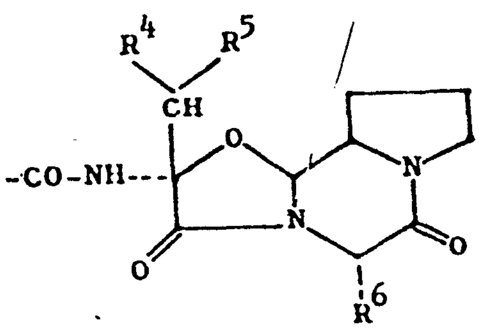

R' and R" each independently is H or $C_{1-6}$-alkyl, $R^4$ and $R^5$ each independently is hydrogen or methyl, $R^6$ is phenylmethyl, isobutyl, or isopropyl and $C_9$----$C_{10}$ is a CC single or double bond, or a pharmacologically acceptable salt thereof.

4. A composition of claim 3, further comprising a pharmacologically acceptable carrier.

5. A composition of claim 2, comprising 0.01-50 mg of ergot alkaloid and 25-1000 mg of L-dopa.

6. A composition of claim 2 adapted for oral administration.

7. A composition of claim 1, wherein the ergot alkaloid is lisuride hydrogen maleate, 1,1-diethyl-3-(6-methyl-8α-ergolinyl)urea, 1,1-diethyl-3-(2-bromo-6-methyl-8α-ergolinyl)urea, 1,1-diethyl-3-(2-bromo-6-methyl-9,10-didehydro-8α-ergolinyl)urea, 1,1-diethyl-3-3-(9,10-didehydro-6-n-propyl-8α-ergolinyl)urea, 1,1-diethyl-3-(6-n-propyl-8α-ergolinyl)urea, pergolide, lergotril, bromocriptine, or ergotamine.

8. A composition of claim 1, wherein the ergot alkaloid is lisuride.

9. A method of inhibiting the growth of malignant cells in a patient in need of such treatment, comprising administering to the patient a cytostatically effective amount of a composition of claim 1.

10. A method of claim 9, wherein the malignant cells are those of human leukemia or a solid human tumor.

11. A method if claim 9, wherein the malignant cells are those of human leukemia, lung carcinoma, heart carcinoma, thyroid carcinoma, adrenal gland carcinoma or carcinoma of the bronchi, or neuroblastomas.

12. A method of claim 9, wherein the administration continues for a period of more than 3 months.

13. A method of claim 9, wherein the patient is one unable to tolerate anemia, hemorrhaging tendency, or suppression of the immune system as a side effect.

14. A method of claim 9, wherein the ergot alkaloid and L-dopa are administered in unitary form.

15. A method of claim 9, wherein the ergot alkaloid and L-dopa are administered separately.

16. A method of claim 9, wherein the amount of ergot alkaloid is 0.01-50 mg/day and the amount of L-dopa is 25-1,000 mg/day, when administered orally.

17. A method of claim 9, wherein the amount of ergot alkaloid is 0,15 - 1,5 mg/day and the amount of L-dopa is 10-500 mg/day, when administered parenterally.

European Patent Office

**PARTIAL EUROPEAN SEARCH REPORT**

which under Rule 45 of the European Patent Convention shall be considered, for the purposes of subsequent proceedings, as the European search report

0114563

Application number

EP 83 73 0119

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | CHEMICAL ABSTRACTS, vol. 97, no. 13, September 27, 1982, page 56, column 2, ref. 104170z Columbus, Ohio, US S. SASSA et al.: "Effects of perphenazine, metoclopramide, l-dopa, and ergot alkaloids on serum prolactin level in male rats" & Igaku to Seibutsugaku 1982, 104(3), 163-5<br>* Abstract * | 1-8 | A 61 K 31/48// (A 61 K 31/48, 31/195) |
| | -- | | |
| A | EP - A - 0 005 074 (MASSACHUSETTS INSTITUTE OF TECHNOLOGY)<br>* Page 9, lines 1-15, claims 1,2 * | 1-8 | |
| | -- | | TECHNICAL FIELDS SEARCHED (Int. Cl. 3) |
| A | US - A - 3 849 562 (BRIAN PETER RICHARDSON) | | A 61 K |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-8

Claims searched incompletely:

Claims not searched: 9-17

Reason for the limitation of the search: Method for treatment of the human or animal body by surgery or therapy (see article 52(4) of the European Patent Convention)

| Place of search | Date of completion of the search | Examiner |
|---|---|---|
| The Hague | 27-03-1984 | BRINKMANN |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1505.1. 03.82

European Patent Office

# PARTIAL EUROPEAN SEARCH REPORT



| DOCUMENTS CONSIDERED TO BE RELEVANT | | | CLASSIFICATION OF THE APPLICATION (Int. Cl.3) |
|---|---|---|---|
| Category | Citation of document with indication, where appropriate, of relevant passages | Relevant to claim | |
| | * Column 1, line 10 - column 2, line 13 *<br>----- | | |
| | | | TECHNICAL FIELDS SEARCHED (Int. Cl.3) |